# EUROPEAN PATENT APPLICATION

(11) **EP 1 600 435 A1**
(43) Date of publication of application: **30.11.2005**
(21) Application number: 04716335.7
(22) Date of filing: 02.03.2004
(51) Int. Cl.: C07C 51/15, C07C 65/03

(54) **PROCESS FOR PRODUCTION OF HYDROXYBENZOIC ACIDS**

(30) Priority: 04.03.2003 JP 2003057260
(71) Applicant: Kabushiki Kaisha Ueno Seiyaku Oyo Kenkyujo, Osaka-shi, Osaka 541-0043 (JP)
(72) Inventor: UENO, Ryuzo, Nishinomiya-shi, Hyogo6620038 (JP); KITAYAMA, Masaya, Takarazuka-shi, Hyogo 6650881 (JP); OTSUKA, Ryoichi, Nishinomiya-shi, Hyogo 6620011 (JP); SHIRAI, Takeshi, Amagasaki-shi, Hyogo 6610951 (JP)
(74) Representative: Albrecht, Thomas
(86) International application number: PCT/JP2004/002554
(87) International publication number: WO 2004/078693

(57) **Abstract**

The present invention provides A method for producing a hydroxybenzoic acid compound comprising, preparing an alkali metal salt of a phenol compound from the phenol compound, and reacting the alkali metal salt of the phenol compound with carbon dioxide, wherein the step of preparing the alkali metal salt of the phenol compound from the phenol compound comprises the steps of:
a) reacting an alkali metal alkoxide with an excess amount of the phenol compound, which is in excess of the alkali metal alkoxide, to give the alkali metal salt of the phenol compound, and
b) distilling away the generated alcohol from the reaction simultaneously with carrying out step a). The method of the present invention makes it possible to produce hydroxybenzoic acid compound in high yield without using aprotic polar organic solvent.

## Description

### TECHINICAL FIELD

The present invention relates to a method for producing a hydroxybenzoic acid compound.

### BACKGROUND OF THE INVENTION

Hydroxybenzoic acid compound having substituents such as alkyl group is used for manufacturing wide variety of materials, for example ultraviolet absorber and antioxidant for polymer materials such as polypropylene, developer for pressure-sensitive recording paper as well as agricultural chemicals.

As a method for preparing a hydroxybenzoic acid compound, Kolbe-Schmitt reaction, which comprises reacting an alkali metal salt of a phenol compound with carbon dioxide has been conventionally used. The starting material of this reaction, an alkali metal salt of a phenol compound, is conventionally prepared by using alkali metal compounds such as alkali metal hydroxide, alkali metal carbonate and the like. The reaction of the phenol compound and the alkali metal compound causes generation of water, and the water inhibits the Kolbe-Schmitt reaction and reduces the yield of the hydroxybenzoic acid compound. Water contained in the alkali metal salt of the phenol compound is difficult to remove efficiently and completely. Accordingly, various means for removing water generated in the preparation of an alkali metal salt of the phenol compound have been proposed.

For example, Japanese Patent Application Laid Open No. Sho 62-61949 discloses a method comprising: mixing 2,4-di-tert-butylphenol with sodium hydroxide, conducting dehydration by heating the mixture under reduced pressure, and reacting thus obtained solid-form sodium salt of 2,4-di-tert-butylphenol with carbon dioxide. Japanese Patent Application Laid Open No. Hei 3-178947 discloses a method comprising: reacting 2,4-di-alkylphenol with an alkali metal hydroxide in the presence of a lower alcohol, then distilling away the alcohol and water from the system and reacting thus obtained anhydrous alkali metal salt of 2,4-di-alkylphenol with carbon dioxide.

In these methods, dehydration of alkali metal salt of phenol compound is conducted in solid phase and takes a pretty long time. The removal cannot completely be removed from the system. In addition, reaction of an alkali metal salt of phenol with carbon dioxide has some problems such as the long reaction time which is required due to the solid-gas phase reaction, the great loss of the reaction material because of side reaction due to the thermal non-uniformity of the reaction and the fluctuate of the yield because of the difficulty in controlling the reaction.

An alkali metal salt of phenol can easily be formed in an aqueous solvent using unsubstituted phenol and alkali metal hydroxide. However an alkali metal salt of a phenol compound having a substituent, such as an alkyl group, is hardly generated in aqueous solvent because said substituted phenol compound exhibits less solubility in water and lessdegree of acidity than unsubstituted phenol.

In order to overcome those problems of the solid-gas phase Kolbe-Schmitt reaction, a method wherein the preparation of an alkali metal salt of a phenol compound and the reaction of the alkali metal salt with carbon dioxide are conducted in solvent or as slurry using aprotic polar organic solvent has been proposed.

For example, Japanese Patent Application Laid Open No. Hei 3-90047 discloses a method for preparing 3,5-di-alkyl salicylic acid, comprising: heating 2,4-di-alkylphenol and alkali metal hydroxide in a mixed solvent of hydrocarbon and 1,3-dimethyl-2-imidazolydinone, dehydrating the resulting mixture by means of azeotropic dehydration to give anhydrous alkali metal 2,4-di-alkylphenolate and reacting said metal salt with carbon dioxide in said mixed solvent to give 3,5-di-alkyl salicylic acid.

Though the use of an aprotic polar organic solvent such as 1,3-dimethyl-2-imidazolydinone in the reaction makes it possible to attain high reaction yield of the alkali metal salt of salicylic acid, said method has some problems in collecting the product from the reaction mixture and recovering the solvent. That is, despite of the high reaction yield, alkali metal salt of 3,5-di-alkylsalicylic acid can not be sufficiently collected by crystallization because of the high solubility of the salt to the aprotic polar organic solvent. In addition, aqueous solution of alkali metal salt of 3,5-di-alkylsalicylic acid obtained by the reaction contains a large amount of aprotic polar organic solvent, and in the next aciding out step, the solvent transfers to the filtrate. Accordingly, it is difficult to collect the expensive aprotic polar organic solvent from the reaction system.

In order to solve the above problems, Japanese Patent Application Laid Open No. Hei 10-231271 proposes a method for preparing a hydroxybenzoic acid compound wherein an aprotic polar organic solvent is used as reaction solvent for the reaction between a phenol compound and an alkali metal compound, characterized in that the molar ratio of the phenol compound to the total amount of the alkali metal compound and aprotic polar organic solvent is larger than 1.

However, the yield of hydroxybenzoic acid compound obtained by said method, which uses an excess amount of the phenol compound over the total amount of the alkali metal compound and the aprotic polar organic solvent, is not sufficient. There is also a problem of side products such as a dimer of the phenol compound produced during the Kolbe-Schmitt reaction in the presence of an aprotic polar organic solvent and therefore, it is difficult to obtain high purity hydroxybenzoic acid compound.

Moreover, aprotic polar organic solvents such as 1,3-dimethyl-2-imidazolydinone contain nitrogen and the like and therefore, even a small amount of such solvents in waste solution may cause eutrophication of ocean and lake. Therefore, much effort will be needed to treat the waste liquid when an aprotic polar organic solvent is employed.

Furthermore, the aprotic polar organic solvent is expensive and using the same results in higher cost.

### SUMMARY OF THE INVENTION

An object of the present invention is providing a method for preparing a highly pure hydroxybenzoic acid compound with high yield. Another object of the present invention is providing a method for preparing a hydroxybenzoic acid compound with a simple procedure, without using an expensive aprotic polar organic solvent.

The present invention provides a method for producing a hydroxybenzoic acid compound comprising, preparing an alkali metal salt of a phenol compound from the phenol compound, and reacting the alkali metal salt of the phenol compound with carbon dioxide, wherein the step of preparing the alkali metal salt of the phenol compound from the phenol compound comprises the steps of:
a) reacting an alkali metal alkoxide with an excess amount of the phenol compound, which is in excess of the alkali metal alkoxide, to give the alkali metal salt of the phenol compound, and
b) distilling away the generated alcohol from the reaction simultaneously with carrying out step a).

In the method of the present invention, using an excess amount of the phenol compound over the alkali metal alkoxide promotes production of the alkali metal salt of the phenol compound and consequently, the alkali metal salt of the phenol compound can be obtained in high yield. The remaining phenol compound can be used again as reaction solvent.

The method of the present invention makes it possible to obtain highly pure alkali metal salt of phenol compound without generating water by using an alkali metal alkoxide as an alkali metal compound. Water will inhibit the Kolbe-Schmitt reaction. Accordingly, hydroxybenzoic acid compound can be obtained in high yield.

According to the method of the present invention, in the reaction of a phenol compound with an alkali metal alkoxide, an alcohol corresponding to the alkali metal alkoxide is formed. However, it is easier to distill away the alcohol than water which is a side-product of the reaction of a phenol compound with an alkali metal hydroxide.

### DETAILED DESCRIPTION OF THE PREFERRED EMBODIMENTS

In the present specification and claims, "excess" amount of the phenol compound means that the amount of the phenol compound is two or more molar parts per 1 molar part of the alkali metal alkoxide. In the present invention, the amount of the phenol compound is preferably 2-30 molar parts, more preferably 3-20 molar parts and even more preferably 4-15 molar parts per 1 molar part of the alkali metal alkoxide. In case where the amount of the phenol compound is less than 2 molar parts, the alkali metal salt of the phenol compound will precipitate and interfere with homogenous stirring because of the high viscosity of the reaction mixture. Using more than 30 molar parts of phenol compound is permissible, but it may not bring about better result than using less amount of phenol compound and therefore, it is not economical.

In the method of the present invention, the reaction of a phenol compound with an alkali metal alkoxide is preferably conducted at a temperature of 80-300°C and more preferably at 120-200°C. If the reaction temperature is lower than 80°C, the rate of distilling out the generated alcohol from the system is decreased. On the contrary, if the reaction temperature is higher than 300°C, the phenol compound might be distilled out of the reaction system and the alkali metal salt of the phenol compound might be thermally decomposed due to such a high temperature. Moreover, high reaction temperatures may bring about side reaction.

In the present invention, the phenol compound preferably used for the reaction with an alkali metal alkoxide is presented by general formula (I): 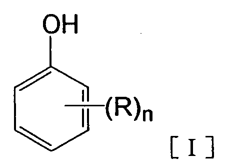 wherein, R is selected from the group consisting of linear or branched chain C1-C20 alkyl, C1-C20 alkenyl and C1-C20 alkoxy groups; n is an integer from 1 to 4.

Among the above, alkyl substituted phenol compound (R=alkyl group), dialkyl substituted phenol compound are suitably used for the method of the present invention. Examples of alkyl groups suitable for the substituents are methyl, ethyl, n-propyl, i-propyl, n-butyl, sec-butyl, tert-butyl, n-octyl and tert-octyl.

Examples of alkyl substituted phenol compound preferably used in the present invention are o-cresol, p-cresol, m-cresol, 2,6-dimethylphenol, 3,5-dimethylphenol, 2,5-dimethylphenol, o-isopropylphenol, 2,6-di-tert-butylphenol, 2,4-di-tert-butylphenol, 2,5-di-tert-butylphenol, 4-n-octylphenol and 4-tert-octyl phenol.

In case there are two or more substituents on the phenol compound, the substituents may be same or different.

A preferable alkali metal alkoxide used in the present invention is sodium C1-C4 alkoxide or potassium C1-C4 alkoxide. In particular, sodium methoxide is preferable because it is easily available and cheap, and the alcohol generated during the reaction with phenol compound can be easily distilled out. An alkali metal alkoxide may be used as solid or in alcohol solution.

In the method of the present invention, alcohol generated during the reaction of the phenol compound with the alkali metal alkoxide or that used as solvent for alkali metal alkoxide is preferably removed from the reaction system until the system contains substantially no alcohol. The removal of alcohol promotes formation of the alkali metal salt of the phenol compound.

The method of removing alcohol is not limited. When the alcohol is removed under atmospheric pressure, it is preferable to remove alcohol by feeding an inert gas such as nitrogen, helium or argon gas, into the reaction mixture of the phenol compound and the alkali metal alkoxide. Alternatively, alcohol may be removed by conducting the reaction under reduced pressure. The pressure of the reaction should be the level where the phenol compound is not vigorously distilled out and may preferably be no lower than 10 Torr.

The gaseous alcohol removed by the above method may be cooled and collected as liquid alcohol. Thus recovered alcohol may be used again as solvent or as starting material, after purifying the same by means of, such as distillation, if necessary.

In the method of the present invention, a substituted phenol, which is in liquid state, is used and said phenol compound acts as solvent. Therefore, it is not necessary to add any other solvent in the step to obtain an alkali metal salt of the phenol compound. However, the case where an additional solvent other than the substituted phenol is used is also included in the scope of the present invention. Solvents other than the phenol compound used in the reaction of the phenol compound with the alkali metal alkoxide may be any solvent other than aprotic polar organic solvent. Examples of such solvents are light oil, kerosene, petrol, white oil, alkylbenzene, alkylnaphthalene, diphenyl, diphenylalkane, alkyldiphenyl, triphenyl, hydrogenated triphenyl, diphenylether, alkylphenylether, alkyldiphenylether, high boiling point higher alcohol such as iso-octyl alcohol and a mixture thereof.

In the method of the present invention, the alkali metal salt of the phenol compound obtained by the reaction of the phenol compound with the alkali metal alkoxide is then reacted with carbon dioxide.

The method of reacting the alkali metal salt of the phenol compound with carbon dioxide is not particularly limited and any conventional method including batch-wise method and continuous method may be employed.

For example, the reaction of the alkali metal salt of the phenol compound with carbon dioxide may be carried out in an autoclave under carbon dioxide pressure of atmospheric pressure to 50.0 kgf/cm²(G), preferably 2.0-10.0 kgf/cm²(G), at a reaction temperature of 160-300°C, preferably of 170-290°C. The reaction time may vary depending on the carbon dioxide pressure and the reaction temperature, and in general, it may be 0.5-6 hrs, and preferably 1-4 hrs.

The reaction mixture of the reaction between the alkali metal salt of the phenol compound and carbon dioxide comprises the corresponding alkali metal salt of benzoic acid compound. Thus obtained reaction mixture was added with water and separated into solvent and aqueous layers. Next, the aqueous layer, i.e. aqueous solution containing the alkali metal salt of the hydroxybenzoic acid compound, is added with an acid to precipitate the hydroxybenzoic acid compound. Non-limiting examples of the acids used for the acid precipitation step include inorganic acids such as hydrochloric acid, sulfuric acid and nitric acid. The precipitates may be harvested by filtration, centrifugation or the like to give crystalline hydroxybenzoic acid compound.

The solvent layer separated from the reaction is mainly consisting of the starting phenol compound and therefore, the solvent layer may be used again as a starting phenol compound directly or, if necessary, after purified by means of, such as, filtration, distillation or carbon treatment.

Non limiting examples of the hydroxybenzoic acid compounds produced by the method of the present invention are 3,5-di-tert-butyl-4-hydroxybenzoic acid, 3,5-di-tert-butyl-2-hydroxybenzoic acid, 3-methyl-2-hydroxybenzoic acid, 3-methyl-4-hydroxybenzoic acid, 3,5-dimethyl-4-hydroxybenzoic acid, 2,6-dimethyl-4-hydroxybenzoic acid, 2-ethyl-4-hydroxybenzoic acid, 3,5-di-ethyl-4-hydroxybenzoic acid, 3-isopropyl-2-hydroxybenzoic acid. Among the above, the present method can preferably be employed in preparing 3,5-di-tert-butyl-4-hydroxybenzoic acid from 2,6-di-tert-butylphenol and 3,5-di-tert-butyl-2-hydroxybenzoic acid from 2,4-di-tert-butylphenol.

According to the method of the present invention, only a low amount of side-products are generated and therefore, highly pure hydroxybenzoic acid compound can be obtained in high yield. Moreover, the method of the present invention does not use an expensive aprotic polar solvent and makes it possible to produce hydroxybenzoic acid compound at low cost with simple steps. Furthermore, the solvent layer separated from the reaction mixture in the method hardly contains by-products and therefore, it can be used again as the starting phenol compound.

The present invention is further described in reference to the following examples.

### Example 1

1155 g (5.6 moles) of 2,6-di-tert-butylphenol and 154 g (0.8 moles) of 28 % sodium methoxide in methanol were fed in 2L stainless-steel vessel equipped with magnetic stirrer, thermometer, pressure gauge and alcohol separator. The reaction mixture was heated to 180°C under nitrogen gas flow. At this temperature, the reaction was conducted for five hours simultaneously with distilling out the by-product, methanol. The resulting slurry of sodium salt of 2,6-di-tert-butylphenol was heated to 210°C and the nitrogen gas in the vessel was replaced with carbon dioxide. Carboxylation was carried under the carbon dioxide pressure of 6 Kgf/cm²(G) with stirring for 2 hours. After the reaction was completed, the reaction mixture was cooled to 90°C and 1200 g of water was added thereto. The obtained mixture was heated to 85°C and the mixture was separated into the aqueous and solvent layers.

To thus obtained aqueous layer, 73 % aqueous sulfuric acid was added to adjust the pH to 3.8 and to precipitate crystal. The crystal was filtrated, washed with water and dried. As a result, 176 g of 3,5-di-tert-butyl-4-hydroxybenzoic acid was obtained. The yield to fed amount of sodium methoxide was 90 %.

### Example 2

To the solvent layer removed from the aqueous layer in example 1, 138 g (0.67 moles) of 2,6-di-tert-butylphenol and 154 g (0.8 moles) of 28 % sodium methoxide in methanol were added. According to the same method with Example 1, 173 g of 3,5-di-tert-butyl-4-hydroxybenzoic acid was obtained. The yield to the fed amount of sodium methoxide was 87 %.

It was confirmed that the excess amount of the phenol compound could be used again as starting material of the method of the present invention.

### Example 3

In the same manner as Example 1 except that the reaction of 2,6-di-tert-butyl phenol with sodium methoxide was conducted at 120°C, 164 g of 3,5-di-tert-butyl-4-hydroxybenzoic acid was obtained. The yield of the fed amount of sodium methoxide was 82 %.

It was confirmed that the hydroxybenzoic acid compound was obtained in high yield even when the reaction was conducted at a relatively low temperature.

### Example 4

966 g (4.7 moles) of 2,4-di-tert-butylphenol and 69.5 g (0.36 moles) of 28 % sodium methoxide in methanol was fed in the same vessel as that used in Example 1. The reaction mixture was heated to 180°C under nitrogen gas flow and at this temperature, the reaction was conducted for 2 hours with distilling out the by-product, methanol.

The resulting slurry of sodium salt of 2,4-di-tert-butylphenol was heated to 200°C and the nitrogen gas in the vessel was replaced with carbon dioxide. The carboxylation was carried under the carbon dioxide pressure of 6 Kgf/cm²(G) with stirring for 2 hours. After the reaction was completed, the reaction mixture was cooled to 90°C and 1000 g of water was added thereto. After that, according to the same procedure as Example 1, 85 g of 3,5-di-tert-butyl-2-hydroxybenzoic acid was obtained. The yield to fed amount of sodium methoxide was 94 %.

### Example 5

984 g (9.1 moles) of o-cresol and 135 g (0.7 moles) of 28 % sodium methoxide in methanol was fed in the same vessel as that used in Example 1. The reaction mixture was heated to 180°C under nitrogen gas flow and at this temperature, the reaction was conducted for 2 hours with distilling out the by-product, methanol.

The resulting slurry of sodium salt of o-cresol was heated to 200°C and the nitrogen gas in the vessel was replaced with carbon dioxide. The carboxylation was carried under the carbon dioxide pressure of 6 Kgf/cm²(G) with stirring for 2 hours. After the reaction was completed, the reaction mixture was cooled to 90°C and 1000 g of water was added thereto. After that, according to the same procedure as Example 1, 91.5 g of 3-methyl-2-hydroxybenzoic acid was obtained. The yield to the fed amount of sodium methoxide was 86 %.

### Example 6

953 g (7 moles) of o-isopropylphenol and 106 g (0.55 moles) of 28 % sodium methoxide in methanol was fed in the same vessel as that used in Example 1. The reaction mixture was heated to 180°C under nitrogen gas flow and at this temperature, the reaction was conducted for 4 hours with distilling out the by-product, methanol.

The resulting slurry of sodium salt of o-isopropylphenol was heated to 200°C and the nitrogen gas in the vessel was replaced with carbon dioxide. The carboxylation was carried under the carbon dioxide pressure of 6 Kgf/cm²(G) with stirring for 2 hours. After the reaction was completed, the reaction mixture was cooled to 90°C and 1000 g of water was added thereto. After that, according to the same procedure as Example 1, 65.4 g of 3-isopropyl-2-hydroxybenzoic acid was obtained. The yield to fed amount of sodium methoxide was 66 %.

### INDUSTRIAL APPLICIABILITY

Hydroxybenzoic acid compound obtained by the method of the present invention can be used for synthesizing a wide variety of materials, for example, ultraviolet absorber and antioxidant for polymer materials such as polypropylene, developer for pressure-sensitive recording paper as well as agricultural chemicals.

## Claims

1. A method for producing a hydroxybenzoic acid compound comprising, preparing an alkali metal salt of a phenol compound from the phenol compound, and reacting the alkali metal salt of the phenol compound with carbon dioxide, wherein the step of preparing the alkali metal salt of the phenol compound from the phenol compound comprises the steps of:
a) reacting an alkali metal alkoxide with an excess amount of the phenol compound, which is in excess of the alkali metal alkoxide, to give the alkali metal salt of the phenol compound, and
b) distilling away the generated alcohol from the reaction simultaneously with carrying out step a).

2. The method of claim 1, wherein the phenol compound and the alkali metal alkoxide are reacted at a temperature of 80-300°C to give the alkali metal salt of the phenol compound.

3. The method of claim 1 or 2, wherein 2-30 parts by mole of the phenol compound is reacted per 1 part by mole of the alkali metal alkoxide to give the alkali metal salt of the phenol compound.

4. The method of any one of claims 1 to 3, wherein the phenol compound is a compound represented by general formula (I): 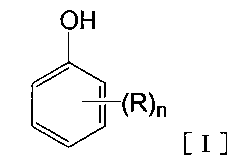 wherein, R is selected from the group consisting of linear or branched C1-C20 alkyl, C1-C20 alkenyl and C1-C20 alkoxy groups; n is an integer from 1 to 4.

5. The method of claim 4, wherein the phenol compound is an alkyl substituted phenol.

6. The method of claim 4, wherein the phenol compound is a di-alkyl substituted phenol.

7. The method of claim 5 or 6, wherein the alkyl moiety of the alkyl substituted phenol is selected from the group consisting of methyl, ethyl, n-propyl, i-propyl, n-butyl, sec-butyl, tert-butyl, n-octyl and tert-octyl.

8. The method of claim 4, wherein the phenol compound is selected from the group consisting of o-cresol, p-cresol, m-cresol, 2,6-dimethylphenol, 3,5-dimethylphenol, 2,5-dimethylphenol, o-isopropylphenol, 2,6-di-tert-butylphenol, 2,4-di-tert-butylphenol, 2,5-di-tert-butylphenol, 4-n-octylphenol and 4-tert-octylphenol.

9. The method of claim 4, wherein the phenol compound is 2,6-di-tert-butylphenol or 2,4-di-tert-butylphenol and the hydroxybenzoic acid compound is 3,5-di-tert-butyl 4-hydroxybenzoic acid or 3,5-di-tert-butyl 2-hydroxybenzoic acid.

10. The method of any one of claims 1 to 9, wherein the alkali metal alkoxide is sodium C1-C4 alkoxide or potassium C1-C4 alkoxide.

11. The method of clam 10, wherein the alkali metal alkoxide is sodium methoxide.

12. The method of any one of claims 1 to 11, wherein any aprotic polar organic solvent is not used as reaction solvent.
